(19) Europäisches Patentamt
European Patent Office
Office européen des brevets

(11) **EP 4 282 316 A1**

(12) **EUROPEAN PATENT APPLICATION**
published in accordance with Art. 153(4) EPC

(43) Date of publication:
**29.11.2023 Bulletin 2023/48**

(21) Application number: **22767539.4**

(22) Date of filing: **10.03.2022**

(51) International Patent Classification (IPC):
**A61B 1/00** (2006.01)          **A61B 1/273** (2006.01)
**G16H 30/40** (2018.01)          **G16H 30/20** (2018.01)
**G16H 50/20** (2018.01)

(52) Cooperative Patent Classification (CPC):
**A61B 1/00; A61B 1/273; G16H 30/20; G16H 30/40; G16H 50/20**

(86) International application number:
**PCT/KR2022/003380**

(87) International publication number:
**WO 2022/191643 (15.09.2022 Gazette 2022/37)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **10.03.2021 KR 20210031339**

(71) Applicant: **Ziovision Co.,Ltd.**
**Chuncheon-si, Gangwon-do 24341 (KR)**

(72) Inventors:
• **PARK, Jung-Whan**
**Chuncheon-si Gangwon-do 24341 (KR)**

• **OH, Jung-Sub**
**Chuncheon-si Gangwon-do 24341 (KR)**
• **KIM, Yoon**
**Chuncheon-si Gangwon-do 24341 (KR)**
• **KIM, Woo-Jin**
**Chuncheon-si Gangwon-do 24341 (KR)**
• **NAM, Seung-Joo**
**Chuncheon-si Gangwon-do 24341 (KR)**

(74) Representative: **Sander, Rolf**
**IPNY AB**
**Birger Jarlsgaten 99A**
**11356 Stockholm (SE)**

(54) **ESOPHAGOGASTRODUODENOSCOPY (EGD) SYSTEM CAPABLE OF ENHANCING RELIABILITY OF EDG INSPECTION USING DEEP LEARNING**

(57) The present invention relates to an esophagogastroduodenoscopy (EGD) system for preventing occurrence of blind spots by checking whether or not examinations have been performed on all positions to be necessarily examined during gastroscopy. The EGD system of the present invention includes a deep learning module for blind spot detection that classifies gastroscopic images obtained by an EGD device according to an anatomical position of a stomach, and a deep learning network of the deep learning module for blind spot detection is a model in which residual learning is used and an attention module is added to a residual block.

[Fig. 1]

**Description**

**[TECHNICAL FIELD]**

The present invention relates to an esophagogastroduodenoscopy (EGD) system capable of improving reliability of gastroscopy using deep learning.

**[BACKGROUND ART]**

Esophagogastroduodenoscopy (EGD) is an examination method for examination of upper gastrointestinal tract diseases such as reflux esophagitis and gastric and duodenal ulcers, and for early detection of gastric cancer.

EGD is to be performed by a specialist recognized by a professional society related to gastroscopy among member societies of the Korean Medical Association. That is, a doctor with such qualifications uses gastroscopy equipment to photograph the subject's stomach, and diagnoses a lesion by observing the photographed image with the naked eye.

For gastrointestinal endoscopy images, technologies such as high definition and white light have already been applied for accurate diagnosis. That is, a hardware part of gastroscopy equipment has sufficient conditions for deep learning technology to be applied, and therefore gastroscopic image analysis research is actively in progress.

According to Lui, Thomas KL, Vivien WM Tsui, and Wai K. Leung. "Accuracy of artificial intelligence-assisted detection of upper GI lesions: a systematic review and meta-analysis.", Gastrointestinal endoscopy Volume 92, Issue 4, pp 821-830, October 2020, results of evaluating the sensitivity of gastroscopy lesion diagnosis by making four groups of deep learning technology, specialists with ten years or more of experience, para-specialists with five years or more of experience, and trainees with less than two years of experience are 94.2%, 94.5%, 85.8%, and 72.2%, respectively, showing that the accuracy when using deep learning technology was almost at the level of specialists with ten years or more of experience. Patent No. 10-2210806 also discloses an apparatus and method for diagnosing gastric lesions in gastroscopic images. As such, the development of deep learning technology for EGD, which has been in progress so far, is mainly focused on diagnosing lesions.

However, diagnosis of lesions using such deep learning technology is performed by analyzing gastroscopic images obtained after normal gastroscopy. When a blind spot occurs due to omission of some regions during gastroscopy, no matter how carefully the gastroscopy is conducted, a problem of missing lesions occurs. Actually, it is known that cases of missing important lesions during gastroscopy due to the blind spot are at about 10%.

As a consequence, in order to increase reliability of gastroscopy, it is important to detect gastric lesions in the gastroscopic image, but more fundamentally, a method capable of preventing blind spots from occurring during gastroscopy is required.

**[DISCLOSURE OF THE INVENTION]**

**[TECHNICAL PROBLEM]**

One object of the present invention is to provide an esophagogastroduodenoscopy (EGD) system capable of preventing blind spots from occurring in a process of gastroscopy.

Meanwhile, other objects not mentioned in the present invention will be additionally considered within the scope to be easily inferred from the following detailed description and effects thereof.

**[TECHNICAL SOLUTION]**

In order to solve the problem described above, an esophagogastroduodenoscopy (EGD) system according to an embodiment of the present invention is provided to prevent blind spots from occurring by checking whether or not examinations have been performed on all positions to be necessarily examined during gastroscopy. According to an embodiment, the EGD system includes a deep learning module for blind spot detection that classifies gastroscopic images obtained by an EGD device according to an anatomical position of a stomach, and a deep learning network of the deep learning module for blind spot detection is a model in which residual learning is used and an attention module is added to a residual block.

According to an embodiment, the deep learning network may learn gastroscopic images of a small intestine, a stomach, and a duodenum as training data and classify the gastroscopic images into classes according to positions of the small intestine, the stomach, and the duodenum.

According to an embodiment, the gastroscopic image of the small intestine or the gastroscopic image of the duodenum may be classified into one class regardless of the anatomical position, and the gastroscopic image of the stomach may be classified into a plurality of classes.

According to an embodiment, the gastroscopic image provided as the training data may include a gastroscopic image in which forceps are photographed, and the gastroscopy with forceps may be classified into a different class from the gastroscopic images of the small intestine, the stomach, and the duodenum.

In order to solve the problem described above, an esophagogastroduodenoscopy (EGD) system according to an embodiment of the present invention includes an EGD device that collects gastroscopic images of an inside of any one of a esophagus, stomach, and duodenum of a subject, a deep learning module for blind spot detection that classifies gastroscopic images obtained by the EGD device according to an anatomical position of a stomach, and an output module that outputs a notification when at least some of positions to be necessarily examined are missing from the gastroscopic images classified by the deep learning module for blind spot detection.

**[EFFECTS OF THE INVENTION]**

An esophagogastroduodenoscopy (EGD) system according to an embodiment of the present invention can improve reliability of gastroscopy by preventing blind spots from occurring in a process of gastroscopy through deep learning.

Even though an effect is not specifically described herein, the effect described in the description below and expected by the technical feature of the present invention and a temporary effect thereof will be considered as being described in the specification of the present invention.

**[BRIEF DESCRIPTION OF THE DRAWINGS]**

FIG. 1 is a schematic configuration diagram of an esophagogastroduodenoscopy (EGD) system according to an embodiment of the present invention.

FIG. 2 is a set of images collected during gastroscopy, showing a representative image for each anatomical position, and FIG. 3 shows the representative images of FIG. 2 as illustrations.

FIG. 4 is a set of images collected during gastroscopy, showing representative images of images to be excluded, and FIG. 5 shows the representative images of FIG. 4 as illustrations.

FIG. 6 is a schematic flowchart of pre-processing steps for extracting only gastric images from images collected during gastroscopy.

FIG. 7 is an image of each pre-processing step, and FIG. 8 shows an image of each pre-processing step of FIG. 7 as an illustration.

FIG. 9 is a schematic architecture of a deep learning model of the EGD system according to an embodiment of the present invention.

FIG. 10 is a reference diagram for describing an SE module used in the deep learning model of the EGD system according to an embodiment of the present invention.

It is clarified that the attached drawings are illustrated as a reference for understanding the technical concept of the present invention, and the scope of the present invention is not limited by the drawings.

**[MODE FOR CARRYING OUT THE INVENTION]**

Hereinafter, with reference to drawings, the configuration of the present invention introduced by various embodiments of the present invention, and the effect caused by the configuration will be described. In describing the present invention, detailed descriptions related to well-known functions and matters obvious to a person skilled in the art will be ruled out when the functions and matters unnecessarily obscures the subject matters of the present invention.

As used herein, the term "module" may include a unit implemented in hardware, software, or firmware, and may interchangeably be used with other terms, for example, "logic," "logic block," "part," or "circuitry". A module may be a single integral component, or a minimum unit or part thereof, adapted to perform one or more functions.

As used herein, "module" or "node" performs tasks such as moving, storing, and converting data using an arithmetic device such as a CPU or AP. For example, a "module" or "node" may be implemented as a device such as a server, PC, tablet PC, or smart phone.

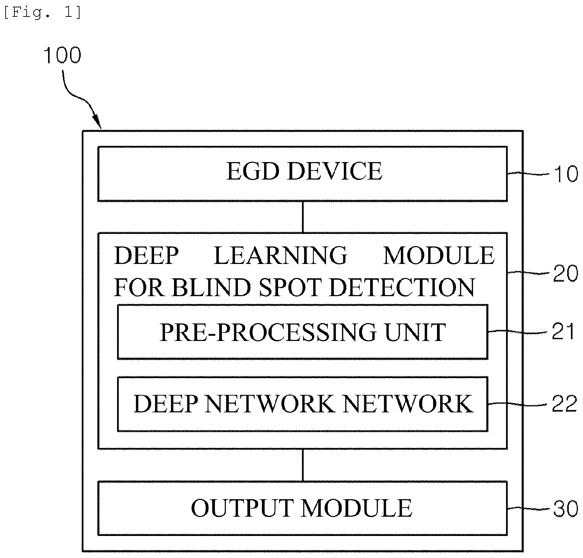

FIG. 1 is a schematic configuration diagram of an esophagogastroduodenoscopy (EGD) system according to an embodiment of the present invention.

Referring to FIG. 1, the EGD system according to an embodiment of the present invention includes an EGD device 10, a deep learning module 20 for blind spot detection, and an output module 30.

The EGD device 10 is a device for performing esophagogastroduodenoscopy (EGD), which is an examination method for the examination of upper gastrointestinal tract diseases such as reflux esophagitis and gastric and duodenal ulcers, and early detection of gastric cancer, and means a device capable of obtaining image information about the esophagus, stomach, and duodenum by inserting an imaging unit into an oral cavity. That is, the EGD device collects gastroscopic images of the inside of any one of the esophagus, stomach, and duodenum of a subject. For the EGD device 10, known devices may be used.

The deep learning module 20 for blind spot detection includes a pre-processing unit 21 for performing pre-processing to learn and determine image information and a deep learning network 22 for analyzing image information transmitted from the EGD device 10 and checking whether or not examinations have been performed on all positions requiring examinations during gastroscopy. Deep learning technology in the related art is used to examine the occurrence of lesions from gastroscopic images, whereas the deep learning module 20 of the EGD system 100 according to an embodiment of the present invention checks whether or not examinations have been performed on all positions to be necessarily examined in a process of gastroscopy, and thus has an effect of preventing blind spots from occurring. The deep learning module 20 for blind spot detection will be described in detail below.

The output module 30 serves to output an operation result of the deep learning module 20 for blind spot detection.

In a first embodiment, in a process of a user performing EGD on a subject by using the EGD device 10, the deep learning module 20 for blind spot detection calculates images transmitted in real time from the EGD device 10, checks whether or not the examinations have been performed at all positions (hereinafter referred to as "essential examination positions") to be necessarily examined in the process of gastroscopy, and informs the user of whether or not a blind spot has occurred in the output module 30 through a display device or a sound device. Due to the anatomical structure of the human body, the EGD has to be performed sequentially from the esophagus to the stomach and duodenum, and the deep learning module 20 for blind spot detection determines whether or not the EGD device 10 has obtained gastroscopic images for the essential examination positions according to a designated order, and when there is a missing part in the order, informs the user of the fact in real time through the output module 30.

In a second embodiment, the user completes an EGD procedure on the subject by using the EGD device 10 and transmits all gastroscopic images to the deep learning module 20 for blind spot detection. The deep learning module 20 for blind spot detection examines the received gastroscopic images to check whether or not gastroscopic images have been obtained for all essential examination positions, and when there are essential examination positions missing, informs the user of the fact through the output module 30.

As described above, when the EGD system of the present invention is used, it is possible to fundamentally prevent blind spots from occurring in the process of gastroscopy, and accordingly, to solve the problem of missing lesions due to blind spots, thereby significantly improving reliability of EGD.

Hereinafter, the deep learning module 20 for blind spot detection of an EGD system 100 of the present invention will be described in more detail with reference to the drawings.

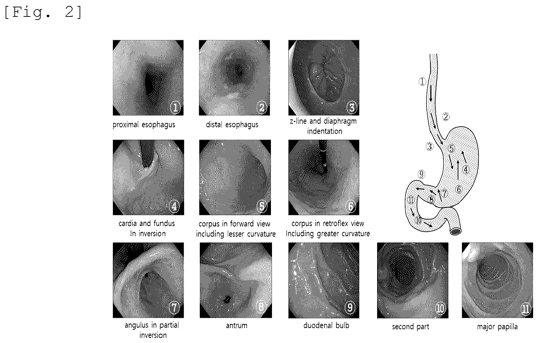

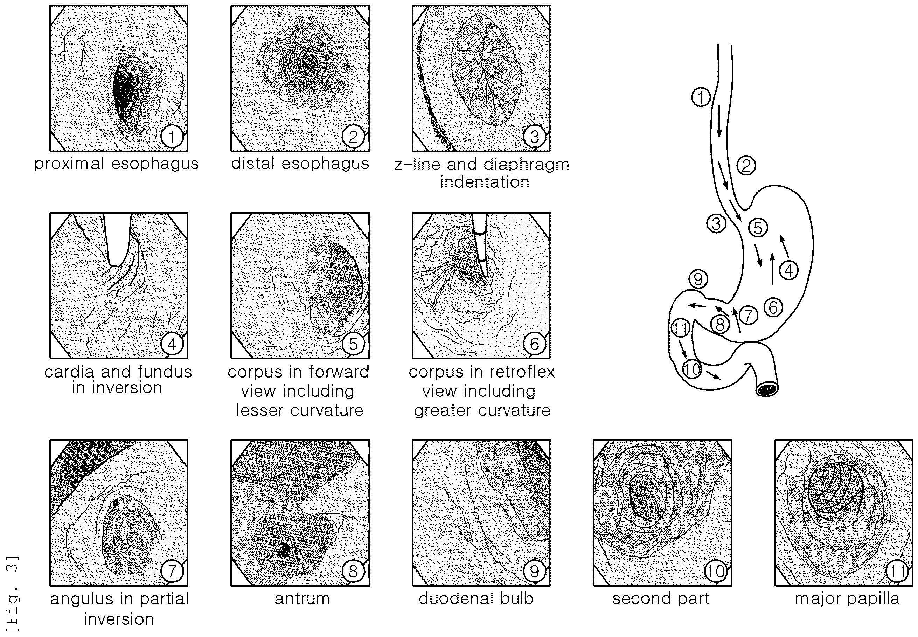

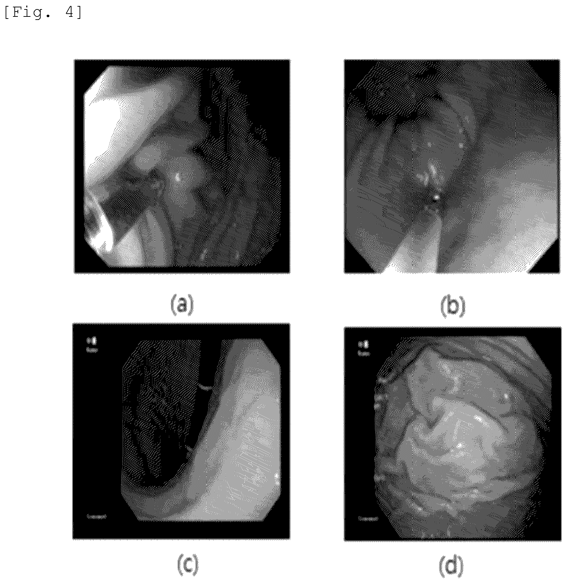

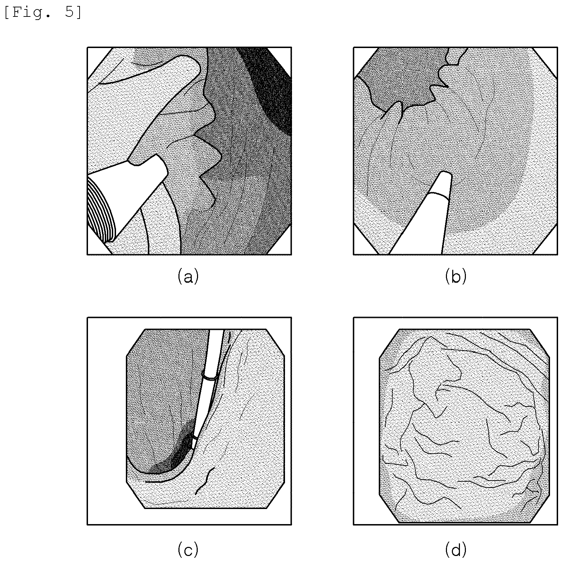

FIG. 2 is a set of images collected during gastroscopy, showing a representative image for each anatomical position, and FIG. 3 shows the representative images of FIG. 2 as illustrations. FIG. 4 is a set of images collected during gastroscopy, showing representative images of images to be excluded, and FIG. 5 shows the representative images of FIG. 4 as illustrations.

In order to train the deep learning module for blind spot detection according to the present invention, 2457 images (Dataset A) for each anatomical position obtained with permission from the Institutional Review Board (IRB) of Kangwon National University Hospital were used (IRB number 2020-07-016-002). Dataset A consists of a total of 2457 gastroscopic images of patients who underwent gastroscopy at Kangwon National University Hospital from January 2015 to November 2020. As shown in FIG. 2, in Dataset A, annotations were registered for each of 11 anatomical positions according to European Society of Gastrointestinal Endoscopy (ESGE) recommendations. However, in the gastroscopic images constituting Dataset A, photographs with forceps as shown in FIGS. 4(a) and 4(b) and images that do not correspond to the 11 anatomical positions according to ESGE recommendations as shown in FIGS. 4(c) and 4(d) are included, and in this case, the images are excluded from the 11 anatomical position classifications. Meanwhile, to verify the deep learning module for blind spot detection, actual gastroscopic images of 60 people with correct answers were also acquired (Dataset B) .

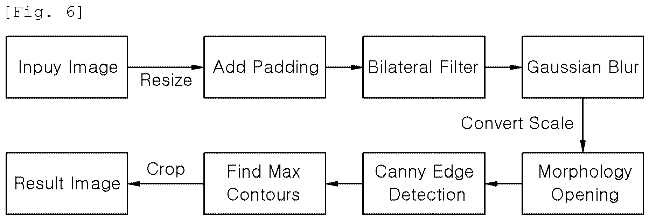

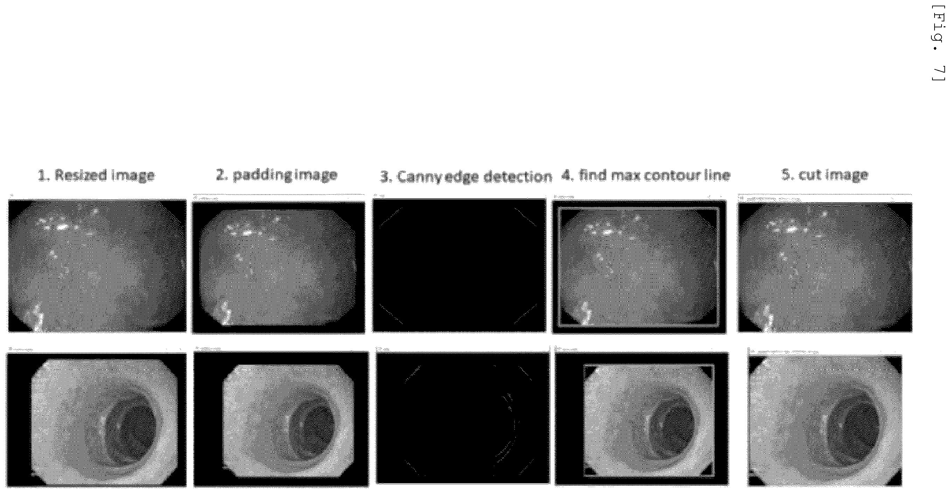

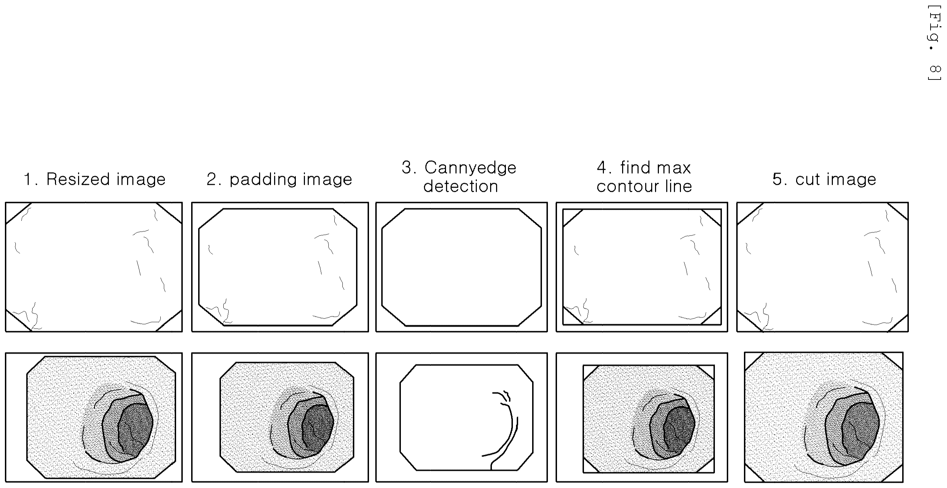

FIG. 6 is a schematic flowchart of pre-processing steps for extracting only gastric images from images collected during gastroscopy. FIG. 7 is an image of each pre-processing step, and FIG. 8 shows an image of each pre-processing step of FIG. 7 as an illustration.

The pre-processing unit of the deep learning module for blind spot detection performs a process of pre-processing the received gastroscopic images and extracting only gastric images. For the EGD device, each image may have a different resolution depending on the type or setting of the device, and text may be included on one side of the gastroscopic image, as shown in FIGS. 4(c) and 4(d). In order to improve the performance of the deep learning network, a pre-processing process of extracting only gastric images from gastroscopic images is required.

The pre-processing unit first performs a step of matching original images having different resolutions to one size. In the present specification, all original images were adjusted to 640 × 480. The pre-processing unit performs a step of adding paddings to the adjusted images to find contours and extract gastric images. The images to which the paddings are added are subjected to a filtering step of using a bilateral filter to make the contours clear and processing a Gaussian blur to remove noise. A step of applying morphology opening to remove text is performed on the images that has been subjected to the filtering step. Then, a step of detecting edges using Canny Edge Detection is performed, and finally, by finding ROIs (Region of Interests) of max contours and cropping the images, only the gastric images from which the text has been removed may be extracted.

The deep learning network of the pre-processing unit of the deep learning module for blind spot detection performs the role of classifying gastroscopic images according to the anatomical positions of the stomach by itself. To this end, the present invention uses a model configured to extract features from the image as the deep learning network and classify the image as an image corresponding to any one of the anatomical positions of the stomach.

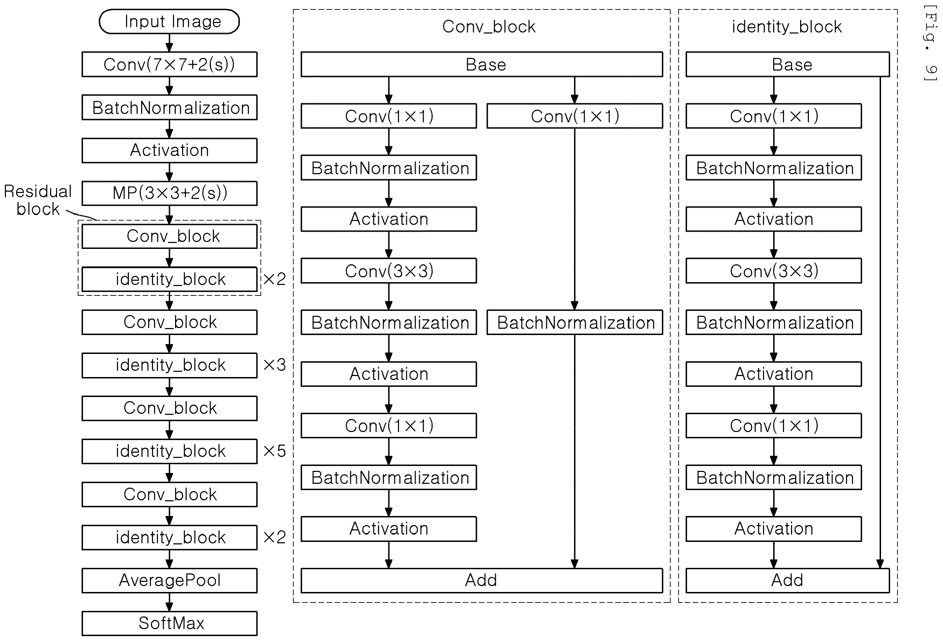

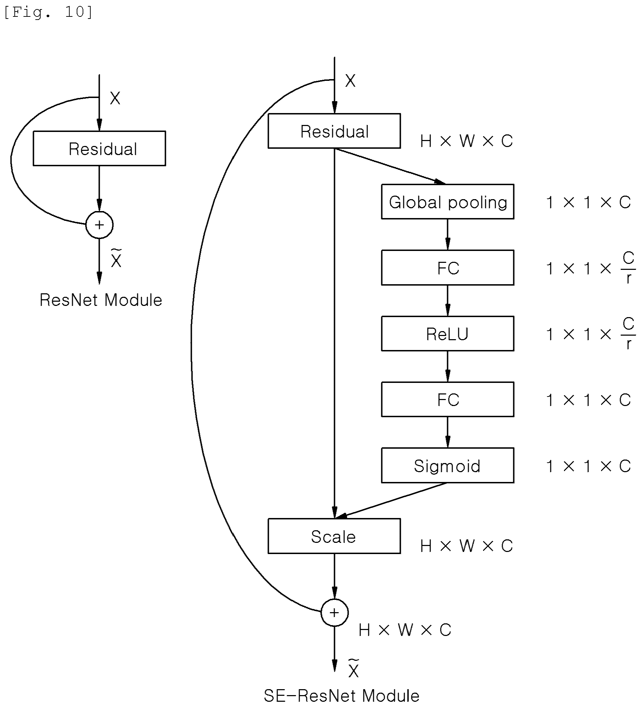

FIG. 9 is a schematic architecture of a model used as the deep learning network of the EGD system according to an embodiment of the present invention, and FIG. 10 is a reference diagram for describing an SE module used in the deep learning model of the EGD system according to an embodiment of the present invention.

Gastroscopic images have similar features even if the anatomical positions are different, and as a consequence, there is a problem that the classification accuracy is not high even if a deep learning network is configured using a method in the related art. In order to solve the problem, the deep learning network according to the present invention uses residual learning, which is configured to preserve previously learned information in a process of training the deep learning network and in addition to that, learn additionally, where an attention module is added to at least one of residual blocks used in a process of the residual learning, and thus there is an effect of remarkably increasing the classification accuracy of gastroscopic images through feature recalibration. For example, Resnet50 may be used as the deep learning network according to the present invention.

As shown in FIG. 9, the residual block means the sum of a conv block and an identity block, and the attention module is added before adding the conv block and the identity block. As the attention module, a squeeze-and-excitation (SE) module may be used. The SE module is configured as shown in FIG. 10, and calculates using a feature map, which is a calculating result of a previous layer (e.g., conv(1×1) + BatchNormalization). The SE module performs calculations to summarize information on feature maps through Squeeze (Global polling + FC), and scales important parts of each feature map through excitation. Specifically, the SE module leaves only important information for each channel by compressing global spatial information about an image for each channel using global average pooling (GAP, which is a method of averaging spatial values corresponding to channels) which is a technique for extracting important information from feature maps from a convolution layer. In addition, the input gastroscopic image has a certain size (for example, 448 × 448 (spatial, space) × 3 (RGB, channel)), and as the size of the input gastroscopic image increases several times or more when it passes through layers of the deep learning network, a space with a value of zero is created, which has an advantage of removing zero values through the SE module.

Meanwhile, since the deep learning network of the EGD system according to an embodiment of the present invention labels gastroscopic images for each of various anatomical positions and classifies the labelled gastroscopic images, binary cross-entropy (BCE) Loss is used as a loss function. Using the sigmoid activation function in a classifier of the deep learning network, a loss of a predicted value of the model is calculated for a correct label. The sigmoid activation function means a function that performs classification by distinguishing an input value as a value between 0 and 1 based on a certain value. The used loss function is shown in Equation (1) below.

[Equation 1]

$$Loss = -\frac{1}{N}\sum_{i=1}^{N_i} t_i \log(y_i) \log + (1-t_i)\log + (1-y_i))$$

In order to train the deep learning network of the present invention, Dataset A is defined as a class as shown in Table 1 below.

[Table 1]

| Organ | Segment | | Class |
|---|---|---|---|
| Esophagus | Proximal Esophagus | | E1 |
| | Distal Esophagus | | E2 |
| | Z-line & Diaphragm Indentation | | E3 |
| Stomach | Cardia and Fundus in Inversion | | S1 |
| | Corpus in Forward View including Lesser Curvature | | S2 |
| | Corpus in Retroflex View including Greater Curvature | | S3 |
| | Angulus in Partial | | S4 |
| | Inversion | | |
| | | Antrum | S5 |
| Duodenal | | Duodenal Bulb | D1 |
| | | Second Part | D2 |
| | | Major Papilla | D3 |

Meanwhile, in the present invention, in order to improve the classification performance of positions of the stomach (S1 to S5), each of subclasses of the esophagus and duodenum is designated as one class. That is, classes E1 to E3 are designated as class E, and classes D1 to D3 are designated as class D. Further, among Dataset A, an image with endoscopic forceps is designated as class C. An experiment was conducted to evaluate the performance of the EGD system according to the present invention and to compare the performance with those of other deep learning network models. The experiment was conducted by dividing Dataset A of 2457 sheets into 2203 sheets of training data and 254 sheets of test data. An experimental method was conducted by dividing a data size into 224 × 224 and 448 × 448, and in the case of a scratch model, in a batch size of 8, 100 epochs were performed, and in the case of using an Imagenet pretrained model, the classifier was modified and in a batch size of 8, 30 epochs were performed. In addition, in the present experiment, the Adam optimization technique was used to improve classification performance as the method proposed in Bag of Tricks [Cubuk, Ekin D., Barret Zoph, Dandelion Mane, Vijay Vasudevan, and Quoc V. Le. "Autoaugment:Learning augmentation policies from data." arXiv preprint arXiv:1805.09501, 2018.], and a learning rate was adjusted for each epoch using cosine annealing decay. For data augmentation, a data augmentation technique proposed by the Google Brain team for CIFAR-10 presented in the method proposed at CVPR 2019, which is an AutoAugment method [He, Tong, Zhi Zhang, Hang Zhang, Zhongyue Zhang, Junyuan Xie, and Mu Li. "Bag of tricks for image classification with convolutional neural networks." In Proceedings of the IEEE Conference on Computer Vision and Pattern Recognition, pp. 558-567.2019.], was used. The AutoAugment method is a reinforcement learning-based method that shows optimal data augmentation for datasets on Imagenet, CIFAR-10, CIFAR-100, Stanford Cars, and SVHN. The Augment method combined 16 methods of Shear X/Y, Translate X/Y, Rotate, Solarize, Cutout, Invert, Equalize, AutoContrast, Contrast, Brightness, Sharpness Sample Pairing, and presented 25 policies, 5 for each of 5 datasets. Among them, the learning used a policy for finding the optimal data augmentation for the CIFAR-10 dataset.

[Table 2]

| Class | Train(N) | Valid(N) |
|---|---|---|
| C | 31 | 4 |
| D | 237 | 30 |
| E | 382 | 49 |
| S1 | 270 | 29 |
| S2 | 328 | 36 |
| S3 | 333 | 36 |
| S4 | 291 | 36 |
| S5 | 331 | 34 |

As can be seen in Table 2, regarding the class imbalance problem, class ratios are considered so that the weights are learned at larger ratios for classes of smaller numbers. An experimental environment is as shown in Table 3 below.

[Table 3]

| OS | Windows 10 Pro |
| --- | --- |
| CPU | Xeon W-2123 |
| GPU | RTX 2080 Ti 11GB |
| RAM | 64GB |
| framework | pytorch |

Performance evaluation for the experiment used Recall, Precision, and F1 Score, which are performance evaluation indicators for multi-label classification and class imbalance data. The evaluation was conducted by obtaining TP (True Positive), FP (False Positive), and FN (False Negative) between predicted values of the model and the correct answer data. Formulas for Recall (Sensitivity), Precision (PPV), and F1 Score are shown in Equations 2 to 4 below.

[Equation 2]

$$(True)Recall = \frac{TP}{TP + FN}$$

[Equation 3]

$$(True)Precision = \frac{TP}{TP + FP}$$

[Equation 4]

$$F1\ Score = \frac{2 \times Precision \times Recall}{Precision + Recall}$$

Table 4 below shows results of evaluating the sensitivity, precision (PPV), and F1 Score of the scratch model trained from the beginning, and Table 5 shows results of evaluating the sensitivity, precision (PPV), and F1 Score of the pretrained model.

[Table 4]

| | Model | Sensitivity | PPV | F1 score |
| --- | --- | --- | --- | --- |
| Comparati ve Example 1 | Resnet 50_224 | 0.97 64 | 0.95 02 | 0.9631 |
| Comparati ve Example 2 | Resnet 50_448 | 0.96 65 | 0.97 23 | 0.9704 |
| Comparati ve Example 3 | Densen et121_224 | 0.99 21 | 0.98 05 | 0.9863 |
| Comparati ve Example 4 | Densen et121_448 | 0.97 24 | 0.96 86 | 0.9705 |
| Comparati ve Example 5 | Effici entnet-B1_224 | 0.97 64 | 0.96 12 | 0.9688 |
| Comparati ve Example 6 | Effici entnet-B1_448 | 0.98 82 | 0.95 80 | 0.9729 |
| Example 1 | SE_Res net50_224 | 0.95 67 | 0.96 81 | 0.9624 |

(continued)

| | Model | Sens itivity | PPV | F1 score |
|---|---|---|---|---|
| Example 2 | SE_Res net50_448 | 0.96 85 | 0.96 47 | 0.9666 |
| Example 3 | CBM_Re snet50_224 | 0.97 24 | 0.98 02 | 0.9763 |
| Example 4 | CBM_Re snet50_448 | 0.97 64 | 0.98 02 | 0.9783 |

[Table 5]

| | Model | Sensit ivity | PPV | F1 score |
|---|---|---|---|---|
| Com parative Example 1 | Resnet 50_224 | 0.9685 | 0.9685 | 0.9685 |
| Com | Resnet | 0.9606 | 0.9760 | 0.9683 |
| parative Example 2 | 50_448 | | | |
| Com parative Example 3 | Densen et121_224 | 0.9803 | 0.9803 | 0.9803 |
| **Com parative Example 4** | **Densen et121_448** | 0.9764 | 0.9920 | **0.9841** |
| **Com parative Example 5** | **Effici entnet-B1_224** | 0.9921 | 0.9921 | **0.9921** |
| **Com parative Example 6** | **Effici entnet-B1_448** | 0.9961 | 0.9961 | **0.9961** |
| **Exa mple 1** | **SE_Res net50_224** | 0.9724 | 1.0 | **0.9880** |
| Exa mple 2 | SE_Res net50_448 | 0.9724 | 0.9880 | 0.9802 |

Table 5, which shows the results of the ImageNet Pretrained model, shows a 0.5 to 2% increase in performance compared to Table 4, which shows the results of the scratch model. In addition, compared to Resnet 50 (Comparative Examples 1 and 2) using residual blocks, in Table 4, Examples 3 and 4 in which the attention module was added, and in Table 5, Examples 1 and 2 in which the attention module was added were 1 to 2% higher in terms of F1 Score. As for the results according to the input size, it is generally known that the larger the size of the input image, the better the model learns the features of the image, which may improve classification performance, but in the domain of gastroscopic images, there were similar features between classes, so the difference according to size was not conspicuous.

For a classification model required for the EGD system to check whether or not a gastroscopy operator has photographed the inside of the stomach without a blind spot, it is important not only to accurately predict classes S1 to S5 defined in Table 1 (sensitivity), but also not to misclassify positions other than S1 to S5.

However, when the model is evaluated in the above experiment, the classification is performed based on a class probability of 50%, which is the output value of the model, and thus, the sensitivity may be high, but there is a possibility that the classification model misclassifies images that it has not seen during learning. Therefore, an additional experiment was conducted for a class probability of 99%, which allows certain images to be classified as correct answers, and practical usability of the present invention was examined through clinical data.

Models to be used for verification are Comparative Examples 3 to 6 and Example 1, which are the top 4 in terms of F1 Score in Table 5, and Dataset B, which is clinical data of 60 people, was used for data. Like Dataset A, Dataset B also consists of the classes defined in Table 1 and the class defining other images as X. In addition, as shown in Table 6, 60 people are divided into 6 groups of 10 people each, one group including all positions of S1 to S5 and 5 groups each missing one position. The experimental method divides models into a class probability of 50% and a class probability of 99% that allows only certain images to be classified as correct answers.

[Table 6]

| Index | Case | N(Person, Image) |
|---|---|---|
| 1 | All | 10, 357 |
| 2 | No S1 | 10, 403 |
| 3 | No S2 | 10, 345 |
| 4 | No S3 | 10, 331 |

(continued)

| Index | Case | N(Person, Image) |
|---|---|---|
| 5 | No S4 | 10, 319 |
| 6 | No S5 | 10, 303 |

The experiment was conducted in a manner suitable for each of group All and the remaining groups. For group All, the experiment was conducted on people for whom the classification model has classified all of S1 to S5, and for the remaining groups, it was checked whether the classification model found a person who was missing at least one of the S1 to S5 classes. Tables 7 to 9 are experimental results of the above method. Table 7 is results obtained by using the class probability condition of 50%, and Table 8 is results obtained by using the condition of 99%. Table 9 shows numbers of people added based on Tables 7 and 8 and accuracies accordingly.

[Table 7]

| Case | Model | | | |
| | Comp arative Example 4 | Compa rative Example 5 | Compar ative Example 6 | Example 1 |
|---|---|---|---|---|
| All | 10 | 10 | 10 | 10 |
| No S1 | 3 | 1 | 3 | 2 |
| No S2 | 0 | 0 | 0 | 2 |
| No S3 | 8 | 8 | 4 | 6 |
| No S4 | 5 | 4 | 4 | 5 |
| No S5 | 6 | 5 | 4 | 6 |

[Table 8]

| Case | Model | | | |
| | Comp arative Example 4 | Compa rative Example 5 | Compar ative Example 6 | Example 1 |
|---|---|---|---|---|
| All | 8 | 9 | 9 | 9 |
| No S1 | 3 | 4 | 3 | 6 |
| No S2 | 1 | 3 | 2 | 3 |
| No S3 | 10 | 10 | 8 | 10 |
| No S4 | 9 | 10 | 7 | 9 |
| No S5 | 8 | 7 | 6 | 8 |

[Table 9]

| Model | Probability | Sum | Accuracy |
|---|---|---|---|
| Compar ative Example 4 | 50% | 32 | 0.533 |
| | 99% | 39 | 0.65 |
| Compar ative Example 5 | 50% | 28 | 0.467 |
| | 99% | 43 | 0.717 |
| Compar ative Example 6 | 50% | 25 | 0.417 |
| | 99% | 35 | 0.583 |

(continued)

| Model | Probability | Sum | Accuracy |
|---|---|---|---|
| Exampl e 1 | 50% | 31 | 0.517 |
| | **99%** | **45** | **0.75** |
| | | | |

As shown in Table 9, when the class probability is 99%, the accuracy is improved by 12 to 25% compared to when the class probability is 50%. In particular, Example 1 shows the highest accuracy by accurately verifying 45 out of 60 people.

The experimental results in Table 5 shows that the sensitivity of Comparative Example 6, the latest deep learning network model, exhibits a performance of 99.61%, but in Table 9 considering clinical trials, the sensitivity of Comparative Example 6 exhibits performances of 41.7% and 58.3%, which are significantly reduced. However, Example 1 exhibits a performance of 100% in the evaluation metric (PPV) where the actual correct answer is true for what the model predicted to be true even though the sensitivity is 97.24% in Table 5, and thus even in Table 8, which is the result of the clinical trials, it can be seen that Example 1 has the highest performances of 51.7% and 75%. That is, it can be seen that the EGD system of the present invention is very suitable for detecting blind spots in gastroscopic images compared to other deep learning networks.

The EGD system as described above may be implemented as a program (or application) including an executable algorithm that may be executed on a computer. The program may be stored in a non-transitory computer readable medium and may be provided. Here, the non-transitory computer readable medium means a machine-readable medium for storing data semi-persistently rather than a medium for storing data temporarily, such as register, cache, and memory. In detail, the above-described various applications and programs may be provided by being stored in a non-transitory readable medium such as a CD, DVD, hard disc, Blu-ray disc, USB, memory card, or ROM.

The scope of the present invention is not limited by the examples and descriptions specifically described so far. Furthermore, it is stated once more that the scope of the present invention should not be construed to be limited by an obvious change or a substitution in the field to which the present invention pertains.

**Claims**

1. An esophagogastroduodenoscopy (EGD) system for preventing occurrence of blind spots by checking whether or not examinations have been performed on all positions to be necessarily examined during gastroscopy, the EGD system comprising:

    a deep learning module for blind spot detection that classifies gastroscopic images obtained by an EGD device according to an anatomical position of a stomach,
    wherein a deep learning network of the deep learning module for blind spot detection is a model in which residual learning is used and an attention module is added to a residual block.

2. The EGD system of claim 1, wherein the deep learning network learns gastroscopic images of a small intestine, a stomach, and a duodenum as training data, and classifies the gastroscopic images into classes according to positions of the small intestine, the stomach, and the duodenum.

3. The EGD system of claim 2, wherein the gastroscopic image of the small intestine or the gastroscopic image of the duodenum is classified into one class regardless of the anatomical position, and the gastroscopic image of the stomach are classified into a plurality of classes.

4. The EGD system of claim 2, wherein the gastroscopic image provided as the training data includes a gastroscopic image in which forceps are photographed, and the gastroscopic image in which forceps are photographed is classified into a different class from the gastroscopic images of the small intestine, the stomach, and the duodenum.

5. An EGD system comprising:

    an EGD device that collects gastroscopic images of an inside of any one of an esophagus, stomach, and duodenum of a subject;

a deep learning module for blind spot detection that classifies gastroscopic images obtained by the EGD device according to an anatomical position of a stomach; and

an output module that outputs a notification when at least some of positions to be necessarily examined are missing from the gastroscopic images classified by the deep learning module for blind spot detection.

[Fig. 1]

100

| EGD DEVICE | 10 |

DEEP LEARNING MODULE
FOR BLIND SPOT DETECTION — 20

| PRE-PROCESSING UNIT | 21 |

| DEEP NETWORK NETWORK | 22 |

| OUTPUT MODULE | 30 |

[Fig. 2]

| | | |
|---|---|---|
| proximal esophagus | distal esophagus | z-line and diaphragm indentation |
| cardia and fundus in inversion | corpus in forward view including lesser curvature | corpus in retroflex view including greater curvature |
| angulus in partial inversion | antrum | duodenal bulb |

second part | major papilla

[Fig. 3]

14

[Fig. 4]

(a)

(b)

(c)

(d)

[Fig. 5]

(a)

(b)

(c)

(d)

[Fig. 6]

| Inpuy Image | →Resize→ | Add Padding | → | Bilateral Filter | → | Gaussian Blur |

Convert Scale

| Result Image | ←Crop← | Find Max Contours | ← | Canny Edge Detection | ← | Morphology Opening |

[Fig. 7]

[Fig. 8]

EP 4 282 316 A1

1. Resized image     2. padding image     3. Cannyedge detection     4. find max contour line     5. cut image

[Fig. 9]

**identity_block**

Base → Conv(1×1) → BatchNormalization → Activation → Conv(3×3) → BatchNormalization → Activation → Conv(1×1) → BatchNormalization → Activation → Add

**Conv_block**

Base → Conv(1×1) → BatchNormalization → (Add)

Base → Conv(1×1) → BatchNormalization → Activation → Conv(3×3) → BatchNormalization → Activation → Conv(1×1) → BatchNormalization → Activation → Add

Input Image → Conv(7×7+2(s)) → BatchNormalization → Activation → MP(3×3+2(s)) → [Conv_block → identity_block] (Residual block) ×2 → Conv_block → identity_block ×3 → Conv_block → identity_block ×5 → Conv_block → identity_block ×2 → AveragePool → SoftMax

[Fig. 10]

X

Residual

+

$\widetilde{X}$

ResNet Module

X

Residual          H × W × C

Global pooling          1 × 1 × C

FC          $1 \times 1 \times \dfrac{C}{r}$

ReLU          $1 \times 1 \times \dfrac{C}{r}$

FC          1 × 1 × C

Sigmoid          1 × 1 × C

Scale          H × W × C

+          H × W × C

$\widetilde{X}$

SE−ResNet Module

## INTERNATIONAL SEARCH REPORT

| International application No. |
|---|
| **PCT/KR2022/003380** |

**A.    CLASSIFICATION OF SUBJECT MATTER**

**A61B 1/00**(2006.01)i; **A61B 1/273**(2006.01)i; **G16H 30/40**(2018.01)i; **G16H 30/20**(2018.01)i; **G16H 50/20**(2018.01)i

According to International Patent Classification (IPC) or to both national classification and IPC

**B.    FIELDS SEARCHED**

Minimum documentation searched (classification system followed by classification symbols)

A61B 1/00(2006.01); A61B 1/04(2006.01); A61B 5/00(2006.01); G06T 5/00(2006.01)

Documentation searched other than minimum documentation to the extent that such documents are included in the fields searched

Korean utility models and applications for utility models: IPC as above
Japanese utility models and applications for utility models: IPC as above

Electronic data base consulted during the international search (name of data base and, where practicable, search terms used)

eKOMPASS (KIPO internal) & keywords: 위내시경 시스템(esophago gastro duodenoscopy), 맹점검사용 딥러닝 모듈(blind point inspection deep learning module), 출력 모듈(output module), 이미지(image), 딥러닝 네트워크(deep learning network), 잔차 학습(residual learning), 잔차 블록(residual block), 어텐션 모듈(attention module)

**C.    DOCUMENTS CONSIDERED TO BE RELEVANT**

| Category* | Citation of document, with indication, where appropriate, of the relevant passages | Relevant to claim No. |
|---|---|---|
| X<br>A | WU, L. et al. A deep neural network improves endoscopic detection of early gastric cancer without blind spots. Endoscopy. 2019, vol. 51, pp. 522-531.<br>    See ABSTRACT; Results; and Discussion. | 5<br>1-4 |
| A | JP 2014-527837 A (EMURA, F. et al.) 23 October 2014 (2014-10-23)<br>    See entire document. | 1-5 |
| A | JP 2000-051149 A (TECHNO SOPHIA CO., LTD.) 22 February 2000 (2000-02-22)<br>    See entire document. | 1-5 |
| A | WO 2020-201772 A1 (OXFORD UNIVERSITY INNOVATION LIMITED) 08 October 2020 (2020-10-08)<br>    See entire document. | 1-5 |

☑ Further documents are listed in the continuation of Box C.       ☑ See patent family annex.

| * | Special categories of cited documents: | "T" | later document published after the international filing date or priority date and not in conflict with the application but cited to understand the principle or theory underlying the invention |
|---|---|---|---|
| "A" | document defining the general state of the art which is not considered to be of particular relevance | | |
| "D" | document cited by the applicant in the international application | "X" | document of particular relevance; the claimed invention cannot be considered novel or cannot be considered to involve an inventive step when the document is taken alone |
| "E" | earlier application or patent but published on or after the international filing date | | |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | "&" | document member of the same patent family |
| "P" | document published prior to the international filing date but later than the priority date claimed | | |

| Date of the actual completion of the international search | Date of mailing of the international search report |
|---|---|
| **07 June 2022** | **07 June 2022** |

| Name and mailing address of the ISA/KR | Authorized officer |
|---|---|
| **Korean Intellectual Property Office**<br>**Government Complex-Daejeon Building 4, 189 Cheongsa-ro, Seo-gu, Daejeon 35208** | |
| Facsimile No. **+82-42-481-8578** | Telephone No. |

Form PCT/ISA/210 (second sheet) (July 2019)

**EP 4 282 316 A1**

<table>
<tr><td colspan="2">INTERNATIONAL SEARCH REPORT</td><td>International application No.<br>**PCT/KR2022/003380**</td></tr>
</table>

**C.      DOCUMENTS CONSIDERED TO BE RELEVANT**

| Category* | Citation of document, with indication, where appropriate, of the relevant passages | Relevant to claim No. |
|---|---|---|
| A | KR 10-2221943 B1 (THE CATHOLIC UNIVERSITY OF KOREA INDUSTRY-ACADEMIC COOPERATION FOUNDATION et al.) 04 March 2021 (2021-03-04)<br>      See entire document. | 1-5 |
| PX | PARK, J. et al. Automatic anatomical classification model of esophagogastroduodenoscopy images using deep convolutional neural networks for guiding endoscopic photodocumentation. 한국컴퓨터정보학회논문지 (Journal of the Korea Society of Computer and Information). 2021 (online publication date: 31 March 2021), vol. 26, no. 3, pp. 19-28.<br>      See abstract; III. The Proposed Scheme; IV. Experiment; and Conclusions. | 1-4 |

Form PCT/ISA/210 (second sheet) (July 2019)

**INTERNATIONAL SEARCH REPORT**
Information on patent family members

International application No.

**PCT/KR2022/003380**

| Patent document cited in search report | | | Publication date (day/month/year) | Patent family member(s) | | | Publication date (day/month/year) |
|---|---|---|---|---|---|---|---|
| JP | 2014-527837 | A | 23 October 2014 | US | 2012-0245415 | A1 | 27 September 2012 |
| | | | | US | 8579800 | B2 | 12 November 2013 |
| | | | | WO | 2012-127428 | A1 | 27 September 2012 |
| JP | 2000-051149 | A | 22 February 2000 | None | | | |
| WO | 2020-201772 | A1 | 08 October 2020 | EP | 3948770 | A1 | 09 February 2022 |
| KR | 10-2221943 | B1 | 04 March 2021 | KR | 10-2020-0094565 | A | 07 August 2020 |

Form PCT/ISA/210 (patent family annex) (July 2019)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 102210806 A

**Non-patent literature cited in the description**

- **LUI ; THOMAS KL ; VIVIEN WM TSUI ; WAI K. LE- UNG.** Accuracy of artificial intelligence-assisted detection of upper GI lesions: a systematic review and meta-analysis. *Gastrointestinal endoscopy,* October 2020, vol. 92 (4), 821-830
- **CUBUK, EKIN D. ; BARRET ZOPH ; DANDELION MANE ; VIJAY VASUDEVAN ; QUOC V. LE.** Auto- augment:Learning augmentation policies from data. *arXiv,* 2018
- **HE, TONG ; ZHI ZHANG ; HANG ZHANG ; ZHONGYUE ZHANG ; JUNYUAN XIE ; MU LI.** Bag of tricks for image classification with convolutional neural networks. *Proceedings of the IEEE Conference on Computer Vision and Pattern Recognition,* 2019, 558-567